# EUROPEAN PATENT SPECIFICATION

(11) **EP 4 146 316 B1**
(45) Date of publication and mention of the grant of the patent: **13.08.2025**
(21) Application number: 21724277.5
(22) Date of filing: 06.05.2021
(51) Int. Cl.: A61M 16/06, A61M 16/08

(54) **RESPIRATORY INTERFACE DEVICE AND KIT**
BEATMUNGSINTERFACEVORRICHTUNG UND KIT
DISPOSITIF INTERFACE RESPIRATOIRE ET KIT

(30) Priority: 06.05.2020 GB 202006728
(43) Date of publication of application: 15.03.2023
(60) Divisional application: 25188513.3
(73) Proprietor: Intersurgical AG, 9490 Vaduz (LI)
(72) Inventor: PAYNE, Simon Robert, Wokingham Berkshire RG41 2RZ (GB); BOWSHER, Richard Francis, Wokingham Berkshire RG41 2RZ (GB); BOTTOM, David Simon, Wokingham Berkshire RG41 2RZ (GB); GONZALEZ, Emma Diaz, Wokingham Berkshire RG41 2RZ (GB)
(74) Representative: Adamson Jones
(86) International application number: PCT/EP2021/062063
(87) International publication number: WO 2021/224420

(56) References cited:
- EP-A1- 2 140 902
- WO-A1-2011/062510
- WO-A1-2011/110961
- WO-A1-2014/183167
- WO-A1-2019/235939
- GB-A- 2 465 689
- US-A1- 2007 000 492
- US-A1- 2015 122 685

## Description

### FIELD OF THE INVENTION

The present invention relates to a respiratory interface device for providing a flow of respiratory gases to a patient, to components thereof, and to a respiratory interface device kit.

### BACKGROUND TO THE INVENTION

High flow nasal therapy is indicated in the improvement of respiratory efficiency in patients with mild to moderate respiratory distress.

In high flow nasal therapy, respiratory interfaces such as nasal cannulae can be used to deliver respiratory gases to a patient. Typically, a nasal cannula comprises a cannula body having a pair of nasal prongs for delivering respiratory gases into the nostrils of the patient, and side arms for connecting the cannula body to a head strap for securing the device to a patient. The respiratory gases are supplied through an opening in the cannula body, via a conduit connected to a gases supply tube.

Many commercially available respiratory interface devices comprise simple silicone tubes that are relatively soft and malleable. The cannula body, nasal prongs, and side arms may all be made from silicone. One issue with such devices is that the weight of the gases supply tube can pull on the interface, causing it to deform and/or become detached from the patient. In some cases, a clip may be used to fix the gases supply tube to headgear such as elastic head straps, in order to reduce the weight of the tube on the interface itself. Another issue is that movement or manipulation of headgear, for example when head straps are tightened, can cause movement in the cannula body and nasal prongs. For example, the nasal prongs could be squashed, affecting the velocity of the gases passing through the prongs and leading to discomfort. As any movement of the nasal prongs in use can result in irritation, stability of such interfaces is an important consideration in their use.

Most commonly in high flow nasal therapy, a flow of respiratory gases is supplied to a respiratory interface horizontally from a single side of the interface. In some arrangements, it is possible to disconnect the gases supply from one side of the interface and reconnect it at the other side; this for example gives adaptability if the patient moves position when sleeping or eating, or is being attended to by a healthcare professional, and allows movement between beds with gases supply units on different sides.

Various respiratory devices have been proposed in the art.

For example, EP 1 481 702 discloses a nasal cannula assembly having a single horizontal side gases entry. The assembly comprises a face mount part including at least one nasal prong and a removable gases flow manifold having a single horizontal side gases entry which in use is in fluid communication with a gases transport means. The gases transport means may extend from either of the right and left sides of the nasal cannula assembly and is interchangeable between the two by removing the gases flow manifold from the face mount part and re-attaching it in the opposite orientation. A tie or lanyard is used to transfer the weight of a gases supply conduit to the neck of a patient.

WO 2011/062510 discloses a range of respiratory interfaces. Various aspects and embodiments are described. In one described aspect, this document discloses a nasal interface comprising a pliable body having an inlet for connection to a conduit providing a flow of gases and at least one nasal outlet, and a more rigid frame attached to or integrally formed with the body and adapted to engage the conduit and secure the conduit relative to the body. The conduit may connect to the body from below the body and extend downwards from the cannula. In alternatives, a conduit may be provided to one side of a nasal cannula, connecting with a side arm lumen, or two conduits may be provided, one to each side of the cannula, connecting with a respective side arm lumen.

WO 2015/193833 relates to a patient interface and component parts. In one aspect, the patient interface comprises a frame section comprising a gases chamber and a nasal delivery element, and a manifold rotatably secured to the frame section, the manifold comprising an axle structure and being configured to rotate relative to the frame section.

WO 2011/110961 A1 discloses a patient interface device including a support member, a seal member including nasal prongs, and a conduit coupling member. The conduit coupling member includes a connecting portion that inserts into an opening in a central portion of the support member.

It is an object of the present invention to provide an improved nasal interface device for providing a flow of respiratory gases to a patient.

### SUMMARY OF THE INVENTION

According to the invention, there is provided a respiratory interface device according to claim 1, a respiratory interface device kit according to claim 16, and a respiratory interface device according to claim 17. The dependent claims define embodiments of the invention as claimed.

In a first aspect (corresponding to the respiratory device according to claim 1), the present disclosure provides a respiratory interface device comprising a body, a frame and an inlet conduit, wherein:
the frame comprises: a front portion having a front wall and two side walls; side arms; and a support platform extending rearwardly from said front wall, the support platform having an inlet opening formed therein;
the body comprises at least one nasal outlet for interfacing with a user's nostril, in use; a base opening; and at least one opening through which the support platform extends; and wherein
the inlet conduit comprises a connector at a first end that extends through the base opening of the body and the inlet opening of the support platform, and clamps the base wall of the body and the support platform together, a chamber being defined between the support platform and the body, which chamber provides fluid communication between the inlet conduit and the one or more nasal outlets.

In a second aspect (not claimed), the present disclosure provides a respiratory interface device comprising a body, a frame and an inlet conduit, wherein:
the frame comprises: a front portion having a front wall and two side walls; side arms; and a support platform extending rearwardly from said front wall, the support platform having an inlet opening formed therein through which a first end of the inlet conduit is received;
the body comprises at least one nasal outlet for interfacing with a user's nostril, in use; a base opening; and at least one opening through which the support platform extends; a chamber being defined between the support platform and the body, which chamber provides fluid communication between the inlet conduit and the one or more nasal outlets; and wherein
the side walls of the front portion of the frame are separated from the platform.

In a third aspect (not claimed), the present disclosure provides a respiratory interface device comprising a body, a frame and an inlet conduit, wherein:
the frame comprises: two side walls; side arms, and a platform extending between said two side walls, said platform having an inlet opening formed therein;
the body comprises at least one nasal outlet for interfacing with a user's nostril, in use, a base opening, and side openings through which the platform extends; and wherein
the inlet conduit comprises a connector at a first end that extends through the base opening of the body and the inlet opening of the support platform, and clamps the base wall of the body and the support platform together, a chamber being defined between the support platform and the body, which chamber provides fluid communication between the inlet conduit and the one or more nasal outlets.

In a fourth aspect (not claimed), the present disclosure provides a frame for use in a respiratory interface device, wherein
the frame comprises: a front portion having a front wall and two side walls; side arms; and a support platform extending rearwardly from said front wall, the support platform having an inlet opening formed therein; and wherein
the side walls of the front portion of the frame are separated from the platform.

In a fifth aspect (not claimed), the present disclosure provides a conduit for use in a respiratory interface device, said conduit comprising a connector at a first end thereof, wherein
said connector extends upwardly from a main body of the conduit and is adapted for connection with a nasal cannula part to provide a supply of gases thereto, a second end of said conduit being adapted for connection to a gases supply tube.

In a sixth aspect (corresponding to the respiratory interface device kit of claim 16), the present disclosure provides a respiratory interface device kit comprising:
a frame, body, and inlet conduit, as separate parts or fully assembled parts, which parts form, when fully assembled, a respiratory interface device according to the first aspect; and
instructions for assembly and/or use.

### DETAILED DESCRIPTION

### Respiratory Interface Device according to the first and second aspects of the disclosure

The respiratory interface device according to the first aspect of the disclosure comprises a body, a frame and an inlet conduit, wherein the frame comprises a front portion having a front wall and two side walls, side arms, and a support platform extending rearwardly from the front wall, the support platform having an inlet opening formed therein; the body comprises at least one nasal outlet for interfacing with a user's nostril, in use, a base opening, and at least one opening through which the support platform extends; and the inlet conduit comprises a connector at a first end that extends through the base opening of the body and the inlet opening of the support platform, and clamps the base wall of the body and the support platform together, a chamber being defined between the support platform and the body, which chamber provides fluid communication between the inlet conduit and the one or more nasal outlets.

The 'front portion' of the frame is that part of the frame that extends around the front and sides of the platform. The 'side arms' are those parts of the frame that extend to the sides of the front portion and enable the respiratory interface device to be held in place on a patient's face. The side arms may extend from any position on the side walls of the front portion of the frame. Thus, the side arms may continue from the 'ends' of the front portion of the frame, or they may extend from positions on the side walls of the front portion, forward of said ends. In the context of the present application, the 'ends' of the front portion of the frame are those parts of the side walls of the front portion that are, in use, closest to a patient's face. Preferably, the side arms continue from the ends of the front portion of the frame.

In preferred embodiments of the first aspect of the disclosure, the side walls of the front portion of the frame are separated from the platform, and the body comprises a front opening through which the platform extends. In alternative embodiments, the side walls of the front portion of the frame are separated from the platform, and the body comprises side openings through which the platform extends. In further alternative embodiments, the side walls of the front portion of the frame are joined with the support platform, and the body comprises side openings through which the platform extends. The frame may have rounded or chamfered edges.

The respiratory interface device according to the second aspect of the disclosure comprises a body, a frame and an inlet conduit, wherein the frame comprises a front portion having a front wall and two side walls, side arms, and a support platform extending rearwardly from the front wall, the support platform having an inlet opening formed therein through which a first end of the inlet conduit is received; the body comprises at least one nasal outlet for interfacing with a user's nostril, in use, a base opening, and at least one opening through which the support platform extends, a chamber being defined between the support platform and the body, which chamber provides fluid communication between the inlet conduit and the one or more nasal outlets; and the side walls of the front portion of the frame are separated from the platform. As above, the 'front portion' of the frame is that part of the frame the that extends around the front and sides of the platform, and the 'side arms' are those parts of the frame that extend to the sides of the front portion and enable the respiratory interface device to be held in place on a patient's face. As for the respiratory interface device in accordance with the first aspect of the disclosure, the side arms may extend from any position on the side walls of the front portion of the frame; preferably, the side arms continue from the ends of the front portion of the frame. The frame may have rounded or chamfered edges.

In preferred embodiments of the second aspect of the disclosure, the body comprises a front opening through which the platform extends. In alternative embodiments, the body comprises side openings through which the platform extends.

Further embodiments of the first and second aspects of the disclosure are described below. For the avoidance of doubt, unless otherwise indicated, features described in relation to specific embodiments of an aspect of the disclosure may be combined with features described in relation to other specific embodiments in respect of that aspect of the
disclosure.

### Body

The respiratory interface devices in accordance with the first and second aspects of the present disclosure comprise a body, a frame and an inlet conduit. The term 'body' as used herein refers to a body for a nasal cannula, generally comprising base, top, front, rear and side walls, an inlet (opening) for receiving a conduit providing a flow of gases, and at least one nasal outlet provided on the top wall for interfacing with a patient's nostril, in use.

The body comprises at least one opening through which the support platform extends. In some embodiments, the at least one opening through which the support platform extends is a pair of side openings in side walls of the body. In the context of the present application, the term 'extends through' includes an arrangement in which the platform is wholly contained within the body and is located between the two side openings. In preferred embodiments, the at least one opening through which the support platform extends is a front opening in a front wall of the body. (In such preferred embodiments, the side walls of the front portion of the frame are separated from the platform.)

The body preferably comprises a pair of nasal outlets (prongs) for interfacing with a patient's nostrils. The prongs are adapted to fit comfortably within the patient's nostrils and may be curved in shape, for example curving rearwardly and inwardly from their bases, with respect to the front and sides of the body.

The prongs may be of uniform cross-section and/or have a uniform wall thickness along their length. In some embodiments, an area around the base of each prong is thinner than the rest of the body. This may increase flexibility in the prong(s) and dampen the effect of any force acting on the prong(s).

The base opening of the body corresponds with the inlet opening of the platform, such that a first end of the inlet conduit may be received through both openings.

The body may comprise an annular exteriorly raised ring around the base opening. The ring is integrally formed with the body and can distort and/or compress when the inlet conduit is attached to the frame, providing a better seal and thus reduced leakage at this interface between the components. The ring also provides a degree of resistance to rotation, such that, in the assembled device, while the inlet conduit may be attached to the frame in a rotatable manner (as described below), it will not rotate freely when not held in a fixed position on the frame. The annular exteriorly raised ring may comprise means for retaining the inlet conduit in a fixed position on the respiratory interface device while in use. For example, it may comprise one or more recesses for cooperation with a corresponding projection on the inlet conduit, or it may comprise one or more projections for cooperation with a corresponding recess on the inlet conduit.

The rear wall of the body may extend beyond the base thereof, providing an increased area for contact with a patient's upper lip. This may reduce the pressure on the patient and provide improved comfort.

Preferably, the rear wall of the body is shaped to correspond with the shape of a patient's upper lip; thus, it may curve inwardly from its outer edges.

The body is generally made of a pliable material and is preferably transparent. For example, the body may be formed from silicone or a similar material. Desirable properties of a material for the body, as will be understood by a person skilled in the art, include softness and biocompatibility. The material should also be compatible with humidified gases at high oxygen concentrations (up to 100% O₂). Elasticity and structural integrity are further desirable properties in a suitable material.

Transparency in the material of the body is preferred, such that the level of any water collected within the chamber, and any blockages or occlusions, are visible.

The material of the body is preferably suitable for surface printing and/or for the application of a coating or one or more surface finishes, for example to provide patient comfort and for labelling purposes. Suitable surface finishes may include, for example, any of a satin finish, a textured finish, a gloss finish and a patterned finish. Satin may provide the body with a softer feel. Texture may provide comfort or may be used to provide labelling. Gloss may improve visibility for labelling, and provide improved transparency; thus, such a finish may be useful in regions where condensation, etc., is more likely to occur and improved visibility would be useful. A patterned finish may provide decoration but may also be employed to give improved moisture wicking and comfort.

The body may be provided in different sizes, for example with different sized prongs. This allows selection of an appropriate size for an individual patient.

### Frame

The frame of the respiratory interface devices in accordance with the first and second aspects of the present disclosure comprises a front portion, side arms, and a support platform. The front portion has a front wall and two side walls. The support platform extends rearwardly from a front wall of the front portion, and has an inlet opening formed therein. The frame has a single inlet opening, that is, the inlet opening formed in the platform of the frame. The frame may have rounded or chamfered edges.

In some embodiments of the first aspect of the disclosure, the side walls of the front portion of the frame are joined with the support platform and the support platform extends through openings in the side walls of the body. In accordance with the second aspect of the disclosure, and in preferred embodiments of the first aspect of the disclosure, the side walls of the front portion of the frame are separated from the support platform, and the platform extends through a front opening in the body or through side openings in the body.

Particularly when the side walls of the front portion of the frame are separated from the support platform, the respiratory interface device may have improved flexibility, resilience, and springiness, reducing the impact on the cannula body and nasal prongs of a force applied to one or both side arms. For example, movement of a side arm will not necessarily result in an equal degree of movement in the corresponding side wall of the front portion of the frame; and any force applied to a side arm (such as may happen for example when headgear is adjusted), causing movement in said side arm, will not necessarily lead to the transfer of force equally along the side walls of the front portion of the frame and, ultimately, to the front wall, platform, and body of the respiratory interface device. Flexibility of the side arms may facilitate adjustment of the respiratory interface device and improve its fit to the wearer.

The support platform has an inlet opening formed therein, through which the connector of the inlet conduit may be received. The inlet opening is preferably circular in shape, to allow for a rotatable connection with the inlet conduit.

The platform may comprise an annular wall extending upwardly around the inlet opening. Thus, the inlet point of the gases may be above the base of the platform, giving the advantage that any condensate generated in the chamber will be collected below the gases entry point. In this way, 'gurgling', a feature commonly associated with respiratory interface devices, may be reduced. Gurgling can generate a significant amount of noise, which can be disturbing to the patient. In addition to the reduction of gurgling, when the inlet point of the gases is above the base of the platform, any build up of condensate is less likely to result in delivery of a large amount of water to the patient (which may otherwise happen for example when the interface is moved).

The platform extends rearwardly from the front wall of the front portion of frame. Where the side walls of the front portion of the frame are separated from the platform, the platform may take any suitable shape. For example, the platform may firstly extend directly rearwardly from the front wall of the front portion of the frame before widening to follow the contours of the side walls of the front portion of the frame. In this arrangement, the platform may comprise a first, narrower, 'neck' portion and a second, wider, 'shoulder' portion. Alternatively, the platform may extend directly rearwardly from the front wall of the front portion of the frame, or it may extend rearwardly at an angle from the front wall of the front portion of the frame, increasing in width rearwardly.

In some embodiments of the first and second aspects of the disclosure, wherein the platform extends through side openings in the body, the front wall of the front portion of the frame may comprise a recess at either side thereof, to facilitate fitting of the body.

In some embodiments of the first and second aspects of the disclosure, the platform comprises one or more of a forward wall; side walls; and a rear wall. Preferably, the platform lacks a rear wall. In such arrangements, flexibility is maximised in the parts of the body that, in use, come into contact with a patient (i.e., the top and rear of the body).

Where, in accordance with preferred embodiments of the first and second aspects of the disclosure, the platform extends through an opening in a front wall of the body, the platform preferably comprises a forward wall, and more preferably, forward and side walls. The forward wall may be spaced (set back) from the front wall of the front portion of the frame, creating a channel between the forward wall of the platform and the front wall of the front portion of the frame. In this arrangement, a top portion, or lip, of the front wall of the body is accommodated within the channel between the forward wall of the platform and the front wall of the front portion of the frame. The front wall of the body thus seals against the front surface of the forward wall of the platform. The front surface of the forward wall of the platform is a sealing surface. In further preferred embodiments, the body is fitted to forward and side walls of the platform. In this arrangement, the front wall of the body seals against the front surface of the forward wall of the platform and the side walls of the body seal against the outer surfaces of the side walls of the platform. The front surface of the forward wall of the platform and the outer surfaces of the sides walls of the platform are sealing surfaces. Where no forward wall is provided, the front wall of the body sits against the inside surface of the front wall of the front portion of the frame. Where no side walls are provided, the side walls of the body sit against the inside surfaces of the side walls of the front portion of the frame.

Where, in accordance with embodiments of the first and second aspects of the
disclosure, the platform extends through side openings in side walls of the body and the side walls of the front portion of the frame are separated from the platform, the platform comprises side walls. In this arrangement, the body is fitted around the front wall of the front portion of the frame and the side walls of the platform. The outer surface of the front wall of the frame and the outer surfaces of the side walls of the platform are sealing surfaces. The front wall of the front portion of the frame may be provided with a recess at either side thereof, to facilitate fitting of the body, and in this arrangement the side walls of the platform may join with the front wall of the front portion of the frame at positions adjacent the recesses in said front wall such that a continuous sealing surface is created between the outer surface of the front wall of the front portion of the frame and the outer surfaces of the side walls of the platform.

Where, in accordance with embodiments of the first aspect of the disclosure, the side walls of the front portion of the frame are joined with the platform, the platform optionally comprises side walls, which side walls are spaced inwardly from the side walls of the front portion of the frame, creating a channel between each side wall of the platform and the respective side wall of the front portion of the frame. In this arrangement, the body is fitted around the front wall of the front portion of the frame and the side walls of the platform. The outer surface of the front wall of the front portion of the frame and the outer surfaces of the sides walls of the platform are sealing surfaces. As above, the front wall of the front portion of the frame may be provided with a recess at either side thereof, to facilitate fitting of the body, and in this arrangement the side walls of the platform may join with the front wall of the front portion of the frame at positions adjacent the recesses in said front wall such that a continuous sealing surface is created between the outer surface of the front wall of the front portion of the frame and the outer surfaces of the side walls of the platform.

Where, in accordance with embodiments of the first aspect of the disclosure, the side walls of the front portion of the frame are joined with the platform and the platform does not comprise side walls, the body is fitted around the front wall of the front portion of the frame and, in one embodiment, sits against the inside surfaces of the side walls of the front portion of the frame. The front wall of the front portion of the frame may be provided with a recess at either side thereof, to facilitate fitting of the body. In an alternative embodiment in which no side walls are included in the platform, the body is fitted around the front wall of the front portion of the frame and is also fitted around (to the outside surfaces of) the side walls of the front portion of the frame; in this embodiment, the outer surfaces of the front wall and side walls of the front portion of the frame form a continuous sealing surface.

In embodiments in which the platform extends through side openings in side walls of the body, the platform preferably lacks a forward wall.

If present, the rear wall of the platform may be shaped to correspond with the shape of a patient's upper lip, curving inwardly from its outer edges. Where no rear wall is present, a rear edge of the platform may be shaped to correspond with the shape of a patient's upper lip, curving inwardly from its outer edges.

The side arms may be contoured to correspond with the contours of a patient's face. In plan view, the side arms may have a curving shape, curving in a generally convex manner and then in a generally concave manner. In front view, the side arms may dip from the side walls of the front portion of the frame before curving generally upwardly. The side arms may have a greater height (vertical width) than the walls of the first portion of the frame; this may contribute to stability and comfort when fitted to a patient's face. In some embodiments, the side arms may increase in height along their length. The side arms may be thinner (of a lesser depth) than the side walls of the first portion of the frame, especially in embodiments in which the side walls of the first portion of the frame are separated from the platform; this may provide greater flexibility in the side arms, while the thicker side walls of the first portion of the frame may provide some rigidity to the frame.

The side arms may be provided with means for attachment to headgear. For example, a distal end of each side arm may be provided with a pair of slots through which an end of a head strap may be threaded. In contrast with existing devices having side arms constructed from silicone, the respiratory interface device of the present disclosure allows direct attachment of a head strap, without the need for an additional bridging part between the side arms and headgear. In some embodiments, such as when a pair of parallel slots are provided on each side arm, adjustment of a head strap involves the application of a symmetrical force to the head strap; in such embodiments, substantially the same amount of force will be required to tighten and loosen the strap. In other embodiments, the detail of the attachment means may be modified such that non-symmetrical forces may be exerted for the loosening or tightening of a head strap (as may be the case for example when one or more walls of the slots or are angled or chamfered); in such embodiments, it will be easier to either tighten or loosen the strap - for example, it may be desirable to have an easier tightening so as to minimise the time needed to secure the interface to a patient's face, but to have more difficult loosening to ensure no unintended loosening occurs in use.

The respiratory interface devices of the first and second aspects of the disclosure may comprise means for retaining the inlet conduit in a fixed position while in use. Said means may take any suitable form. For example, each side wall of the front portion of the frame may comprise a recess for engagement with a corresponding projection on the inlet conduit, or each side wall of the front portion of the frame may comprise a projection for engagement with a corresponding recess on the inlet conduit. Thus, in use, the inlet conduit may be held in a fixed position on a side wall of the front portion of the frame. Where the body overlies the side walls of the front portion of the frame, such recesses or projections may be provided in side walls of the body. In other examples, retention means such as a recesses or projections may be included on the annular exteriorly raised ring around the base opening of the body, for cooperation with a projection or recess at the first end of the inlet conduit.

The frame provides a rigid structure to the respiratory interface device while maintaining flexibility where needed. The material of the frame should be able to maintain its shape and structure under normal use conditions, without breaking. The frame may be made, for example, from a flexible plastics material. Suitable materials for the frame include polypropylene and polyethylene, which are both thermoplastic synthetic polymers, and more specifically, thermoplastic polyolefins. While polypropylene and polyethylene are preferred materials for the frame, other materials such as other flexible polymeric materials may be used as the material for the frame.

A material having a soft feel may be applied to at least part of the side arms, in order to improve patient comfort. Suitable soft-feel materials may include, for example, thermoplastic elastomers, or textile materials. The soft-feel materials may be applied by any suitable means as will be known by the person skilled in the art.

A one-piece construction of the frame of the present disclosure avoids the need for additional connections or connecting parts in the device, adding to the strength and robustness of the present respiratory interface device.

### Inlet Conduit

The inlet conduit in accordance with the first and second aspects of the disclosure has a connector at a first end, for connecting to the platform of the frame. A second end of the inlet conduit is adapted for connection with a gases supply tube.

Preferably, the inlet conduit is in the form of an elbow, such that the connector extends upwardly from a main body of the conduit at the first end of the conduit.

The connector has a shape complementary to the shape of the inlet opening of the platform and is preferably generally cylindrical in shape. The connector may be provided with means for a permanent snap-fit to the platform; for example, it may be provided with one or more lugs adapted for a snap-fit with the top of the annular wall of the platform.

In some embodiments, three, four, or five or more lugs may be provided. The lugs may be equally spaced from one another. The provision of multiple lugs will avoid or minimise the possibility of too much stress being placed on any single lug. The lug or lugs positioned closest to the second end of the inlet conduit may be thicker than remaining lugs, or otherwise reinforced in comparison thereto. In this way, the lug or lugs that are under the most stress due to any moment exerted on the inlet conduit from the gases supply tube are better adapted to absorb such stress. The lugs may be arranged in any orientation with respect to the first and second ends of the conduit.

The inlet conduit may be made from the same material as the frame, or from another material with enough flexibility for example to enable a snap-fit to the frame.

The inlet conduit may comprise retention means for cooperating with the frame or body, as appropriate, to hold the inlet conduit in a fixed position while in use. The retention means may for example comprise a projection the inlet conduit, for cooperation with a corresponding recess or recesses on the frame or body, or a recess on the inlet conduit, for cooperation with a corresponding projection or projections on the frame or body. In some embodiments, a recess may be provided in each side wall of the front portion of the frame or body, such that the inlet conduit may be fixed in position at either side of the front portion of the frame or body and be rotatable between the two fixed positions.

In preferred embodiments, the inlet conduit is secured to the platform in a rotatable and non-removable manner. A permanent connection of the inlet conduit to the platform contributes to the overall strength of the device, while a rotatable connection of the inlet conduit to the platform allows for the device to be operated in different orientations, without the need for disassembly and reassembly. That is, a 'swivel' connection may be provided between the platform and the inlet conduit, whereby, in use, the inlet conduit may be rotated such the main body of the conduit is moved between left and right sides of the platform. This is advantageous, for example, when the patient changes position when sleeping or eating, or is being attended to by a healthcare professional, or when the side from which the gases supply is provided has to be changed for any reason. It is also advantageous for example when a patient is moved regularly to a prone position, such as is the case for example with patients with COVID-19, as the rotatable connection allows easy repositioning of the gases supply tubing.

As the inlet conduit, and thus the gases supply tube, connects to the rigid frame of the respiratory interface rather than to the body, the weight of the gases supply tube will not cause significant distortion of the body. The weight of the gases supply tube will not lead to disconnection of the supply of gases, which might occur in situations in which there is a connection between a soft, deformable body, and a conduit.

### Respiratory Interface Device according to the third aspect of the disclosure

In accordance with the third aspect of the present disclosure, there is provided a respiratory interface device comprising a body, a frame and an inlet conduit, wherein:
the frame comprises: two side walls; side arms; and a platform extending between said two side walls, said platform having an inlet opening formed therein; the body comprises at least one nasal outlet for interfacing with a user's nostril, in use, a base opening, and side openings through which the platform extends; and wherein
the inlet conduit comprises a connector at a first end that extends through the base opening of the body and the inlet opening of the support platform, and clamps the base wall of the body and the support platform together, a chamber being defined between the support platform and the body, which chamber provides fluid communication between the inlet conduit and the one or more nasal outlets.

For the avoidance of doubt, unless otherwise indicated, features of specific embodiments of this aspect of the disclosure may be combined with features of other specific embodiments of this aspect of the disclosure.

The platform may comprise a rear wall. It does not comprise a forward (front) wall. The platform may also comprise side walls, spaced inwardly from the side walls of the frame, such that a channel is formed between each side wall of the frame and the respective side wall of the platform. Preferably, the platform takes the form of a flat tray extending between the side walls of the frame, i.e., it has no side walls (other than those of the frame). As for various embodiments of the first and second aspects of the disclosure, a rear edge of the support platform, or a rear wall of the platform, may be shaped to correspond with the shape of a patient's upper lip, curving inwardly from its outer edges; the inlet opening may be circular in shape; and the platform may comprise an annular wall extending upwardly around the inlet opening.

The side arms are as described in relation to respiratory interface devices of the first and second aspects of the disclosure.

Suitable materials for the frame are as described above in relation to the first and second aspects of the disclosure, and, as for the first and second aspects, the frame may have rounded or chamfered edges. The side walls of the frame of the respiratory interface device in accordance with this third aspect of the present disclosure correspond to the side walls of the front portion of the frame of the respiratory interface device of the first aspect of the present disclosure wherein said side walls of the front portion of the frame are joined to the platform and may comprise any of the features described in relation thereto.

The body is as described in relation to embodiments of the first and second aspect of the disclosure in which the body has side openings. Where the platform comprises side walls, the body is fitted around said side walls, and the outer surfaces of the side walls of the platform are sealing surfaces. Where the platform lacks side walls, the body is fitted around the side walls of the frame, and the outer surfaces of the side walls of the frame are sealing surfaces.

The inlet conduit is as described in relation to the respiratory interface devices of the first and second aspects of the disclosure.

### Manufacture and Use

The respiratory interface devices of the present disclosure are adapted for use with, or may further comprise, a gases supply tube, such as is commonly used with respiratory interface devices. The gases supply tube may be an unheated, lightweight, flexible tube. Preferably, the tube is transparent to enable detection of occlusion, and reinforced to give resistance to crushing and/or kinking. The gases supply tube may be provided with a clip of any suitable type, such as will be known to a person skilled in the art, for attachment to clothing or bedding, for example. Alternatively, or additionally, a neck-tie or lanyard may be attached to the gases supply tube. The gases supply tube is connected to the supply of gases via a connector that may take any suitable form, as will be well known to a person skilled in the art. The respiratory interface devices of the present disclosure are adapted for use with, or may further comprise, headgear such as head straps, such as are commonly used with respiratory interface devices.

The body, frame and inlet conduit of the devices of the present disclosure may each be prepared by injection moulding and then manually assembled. The elastic properties of silicone ensure a good fit to the frame where silicone is the material of the body, with the connector of the inlet conduit clamping the assembly together. Fitting of the body to the frame involves stretching of the body around the base of the platform and other relevant parts of the frame (e.g., in respect of the first and second aspects of the disclosure, the forward and side walls of the platform, the front wall of the front portion of the frame and the side walls of the platform, or the front and side walls of the front portion of the frame; and in respect of the third aspect of the disclosure, the side walls of the platform or the side walls of the frame) thereby defining a chamber between the platform and the body. Chamfered or rounded edges in the frame may ease fitting of the silicone body thereto. A chamfered or rounded edge in the base opening of the platform may ease insertion of the inlet conduit into the frame.

The devices of the present disclosure may be used in the treatment of hypoxia. The devices are designed to meet or exceed the patient's normal inspiratory demand, creating minimal air dilution and aiming to deliver a higher fraction of inspired oxygen than high concentration non-rebreathing masks, and for longer periods. In comparison to a mask the device also provides increased comfort and enables patients to drink, eat, talk and sleep more easily. High flow therapy provides versatility and continuity of care as patients are weaned off a ventilator, or as their condition becomes more acute. This greater flexibility eliminates the need to switch between oxygen therapy delivery systems. The devices are non-invasive and accurately deliver prescribed oxygen concentrations (up to 100% O2) at flows of 10L to 50L/min, 37°C and 100% relative humidity.

### Frame

In accordance with the fourth aspect of the present disclosure, a frame for use in a respiratory interface device comprises:
a front portion having a front wall and two side walls;
side arms; and
a support platform extending rearwardly from the front wall of said front portion;
the support platform having an inlet opening formed therein; and wherein
the side walls of the front portion of the frame are separated from the platform.

Preferred embodiments of the frame in accordance with this aspect of the disclosure are as described above in relation to the second aspect of the disclosure and as described above in relation to embodiments of the first aspect of the disclosure in which the side walls of the front portion of the frame are separated from the platform.

### Conduit

An inlet conduit as described herein forms a fifth aspect of the present disclosure. Thus, the present disclosure further provides a conduit for use in a respiratory interface device, said conduit comprising a connector at a first end, said connector extending upwardly from a main body of the conduit and being adapted to connect with a nasal cannula part to provide a flow of gases thereto, a second end of said conduit being adapted for connection with a gases supply tube. Preferred features of the conduit are as described herein in relation to the first to third aspects of the disclosure. Most preferably, the conduit is adapted to provide a rotatable, or 'swivel' connection to the platform such that the gases supply tubing may be easily repositioned, in use. For the avoidance of doubt, any feature described herein in relation to the inlet conduit may be combined with any other feature described herein in relation to the inlet conduit.

### Kit

While the device of the present disclosure may be provided fully assembled, the frame, body, and inlet conduit may be provided as separate parts, for assembly by a user. Thus, in accordance with the sixth aspect of the present disclosure, a kit may comprise a frame, body, and inlet conduit as described herein, as separate parts or fully assembled parts, which parts form, when fully assembled, a respiratory interface device according to the first aspect of the present disclosure. and instructions for assembly and/or use. The kit may comprise two or more bodies of different sizes.

### BRIEF DESCRIPTION OF THE DRAWINGS

**Figure 1a** is a front view of a respiratory interface device in accordance with embodiments of the first and second aspects of the present disclosure.
**Figure 1b** is a rear view of a respiratory interface device in accordance with embodiments of the first and second aspects of the present disclosure.
**Figure 2a** illustrates a body having a front opening, according to embodiments of the respiratory interface device of the first and second aspects of the present disclosure.
**Figure 2b** is an alternative view of the body of Figure 2a.
**Figure 3a** is a top view of a frame according to embodiments of the fourth aspect of the present disclosure and in accordance with aspects of the respiratory interface device of the first and second aspects of the present disclosure, wherein the side walls of the front portion of the frame are separated from the platform and the side arms are continuous with the ends of the side walls of the front portion of the frame.
**Figure 3b** is a bottom view of the frame of Figure 3a.
**Figures 4a and 4b** illustrate frames according to embodiments of the respiratory interface device of the first aspect of the present disclosure, wherein the platform and side walls of the front portion of the frame are joined and the side arms are continuous with the ends of the side walls of the front portion of the frame.
**Figure 5** illustrates a body having side openings, according to embodiments of the respiratory interface device of the first, second and third aspects of the present
   disclosure.
**Figure 6** illustrates an assembled respiratory interface device comprising the body of Figure 5 and the frame of Figure 4b.
**Figure 7** is a top view of an inlet conduit according to embodiments of the fifth aspect of the disclosure and according to embodiments of the respiratory interface device of the first, second and third aspects of the present disclosure.
**Figure 8** illustrates a device according to embodiments of the respiratory interface of device of the first and second aspects of the present disclosure, fully assembled and together with a head strap and gases supply tube.

### ILLUSTRATED EMBODIMENTS

The following detailed description is illustrative of devices in accordance with the present disclosure and is not intended as implying specific limitations to the device.

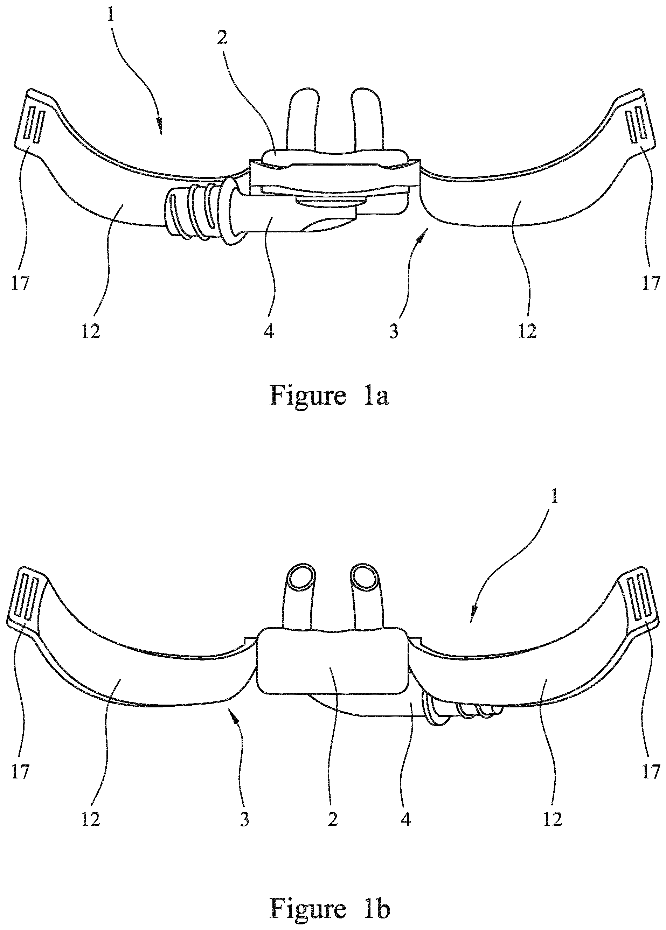

**Figures 1a and 1b** show front (Fig. 1a) and rear (Fig. 1b) views of a fully assembled respiratory interface device in accordance with the present disclosure. In the embodiment shown, the respiratory interface (1) comprises a body (2), a frame (3) and an inlet conduit (4). The body (2) is fitted around the support platform of the frame (3) and clamped thereto by the inlet conduit (4), which is rotatably connected to the support platform of the frame (3). Slots (17) are provided on the distal end of each of the side arms (12) of the frame, to allow attachment of, for example, a head strap.

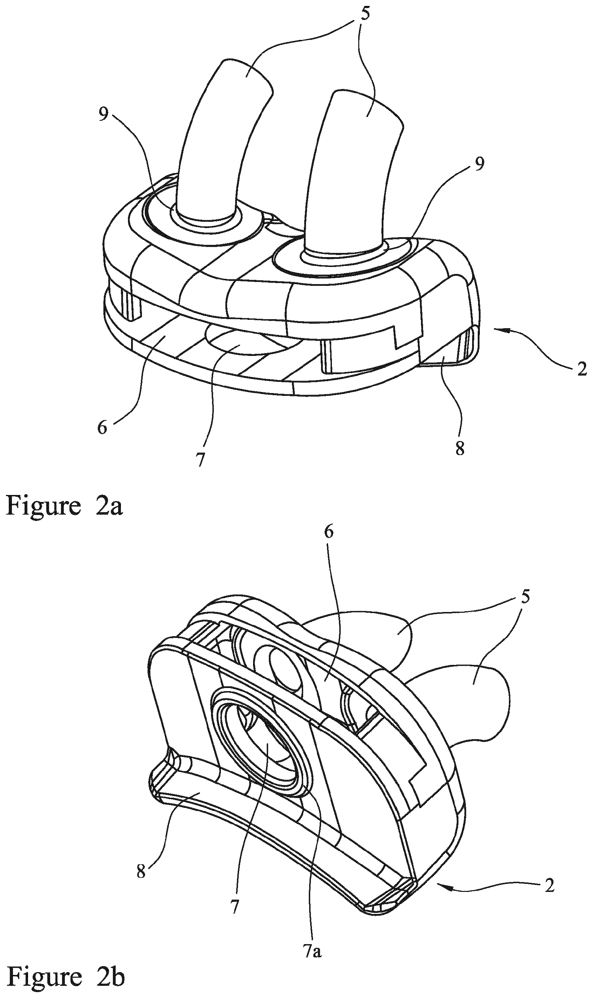

**Figures 2a and 2b** illustrate a body according to the present disclosure. In the embodiment shown, the body (2) comprises two nasal outlets (prongs, 5) for interfacing with a patient's nostrils in use. The body has a front opening (6), a base opening (7), and an annular exteriorly raised ring (7a) around the base opening (7) (as can be seen in Figure 2b).

The body illustrated in Figures 2 and 2b sits to the inside of the front wall of the front portion of the frame in the assembled device, as can be seen in Figure 1a, either against the inside surface of the front wall of the front portion of the frame or in a channel between said front wall and a forward wall of the platform.

In the illustrated embodiment, the prongs (5) curve rearwardly away from the front, and inwardly towards the centre, of the body (2), such that the curve of the prongs is a combination of front and side curves. A rear wall (8) of the body (2) extends beyond the base thereof, to provide an increased contact area between the body and a patient's upper lip. The rear wall (8) curves inwardly from its sides to its centre, thus taking a shape generally corresponding to the shape of a patient's upper lip. Discrete areas (9) of the body around the base of the prongs (5) are thinner than the rest of the body.

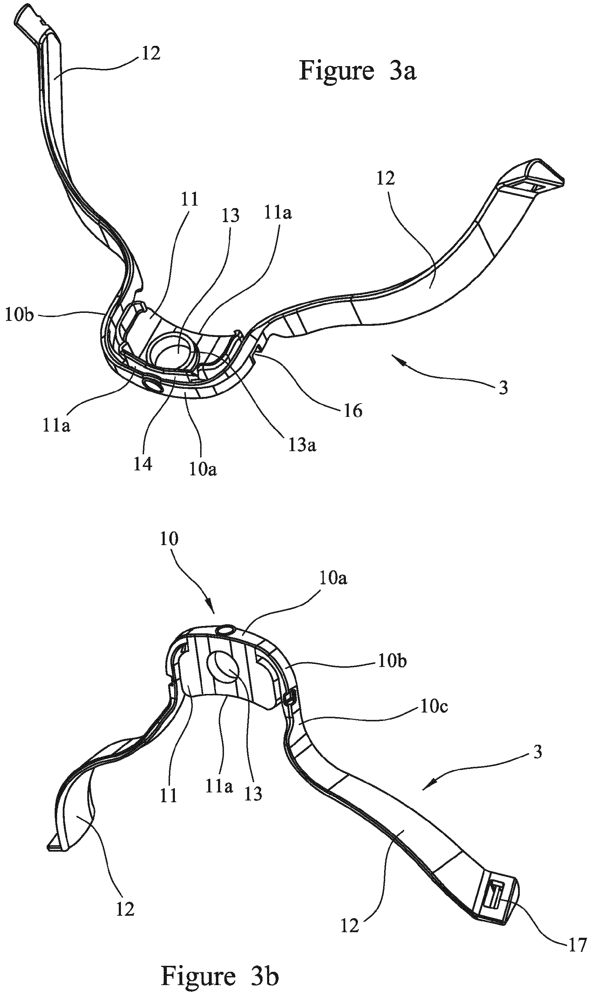

**Figures 3a and 3b** illustrate a frame (3) in accordance with the present disclosure.

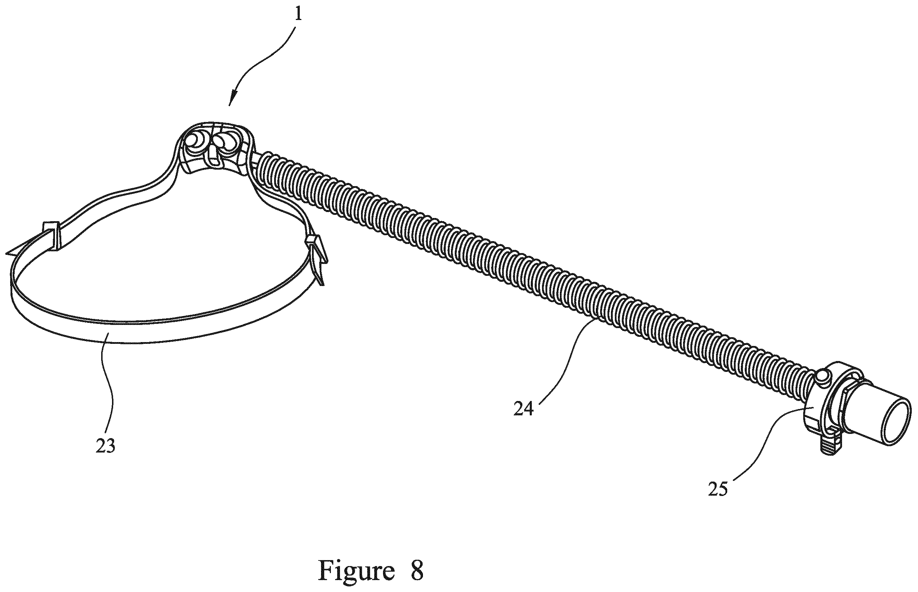

In the embodiment shown, the frame (3) has a front portion (10) comprising a front wall (10a) and side walls (10b), a support platform (11) extending rearwardly from a front wall (10a) of the front portion (10), and side arms (12) extending from each side end (10c) of the front portion (10). The support platform has an inlet opening (13) formed therein. In the illustrated embodiment, the side walls (10b) of the front portion (10) and support platform (11) are separated (spaced apart) from each other. As can be seen from Figure 3b, the platform is continuous with the front wall of the front portion of the frame at the base thereof. A forward wall (14) of the platform (11) is set back from the front wall (10a) of the front portion (10) of the frame (3) to provide a channel between said forward (14) and front (10) walls. Side walls of the platform (11) are spaced apart from the side walls of the front portion of the frame. Thus, the body may be fitted around the platform (11) and clamped thereto by the connector of the inlet conduit. A rear edge (11a) of the platform (11) curves inwardly from its sides to its centre, thus taking a shape generally corresponding to the shape of a patient's upper lip. The inlet opening (13) provided in the base of the platform for connection to an inlet conduit for the delivery of gases is generally circular in shape. The platform (11) further comprises an annular wall (13a) extending upwardly around the opening, to facilitate securement of an inlet conduit. The frame (3) further comprises recesses (16) for locking the inlet conduit into a fixed position on the frame via a projection on the inlet conduit for engagement with said recess. The side arms (12) extend from the ends (10c) of the side walls (10b) of the front portion (10) of the frame (3), and are provided with slots (17) for attachment to headgear (as illustrated, for example, in Figure 8).

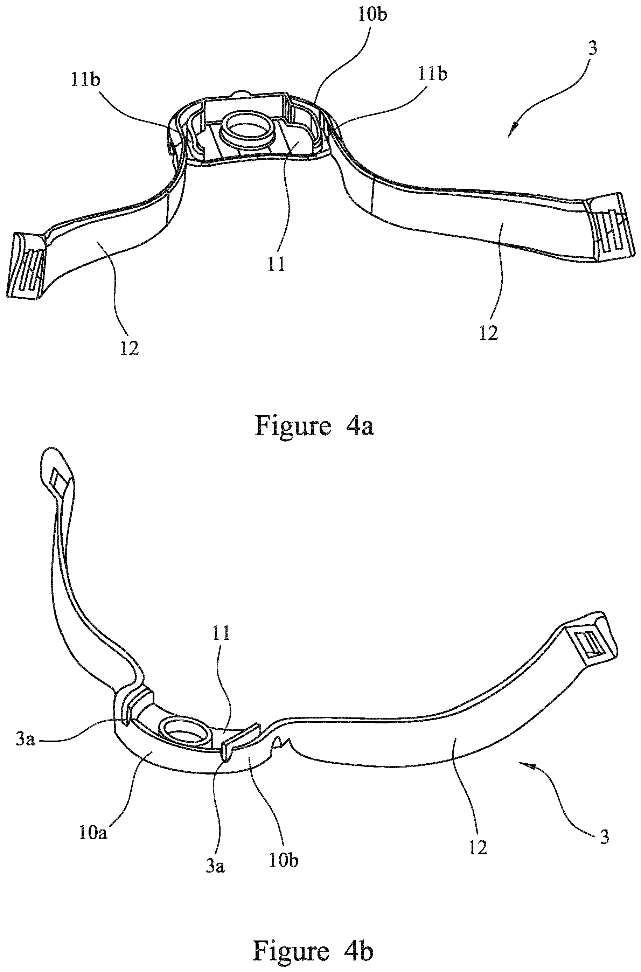

**Figures 4a and 4b** illustrate frames (3) in accordance with embodiments of the disclosure. In the illustrated embodiments, the platform (11) is joined with the side walls (10b) of the front portion (10) of the frame (3). Channels (11b) are formed between the side walls of the platform (11) and the side walls (10b) of the front portion of the frame (10). In the embodiment of Figure 4b, a groove, or recess (3a), is formed at both sides of the front wall (10a) of the front portion (10) of the frame (3).

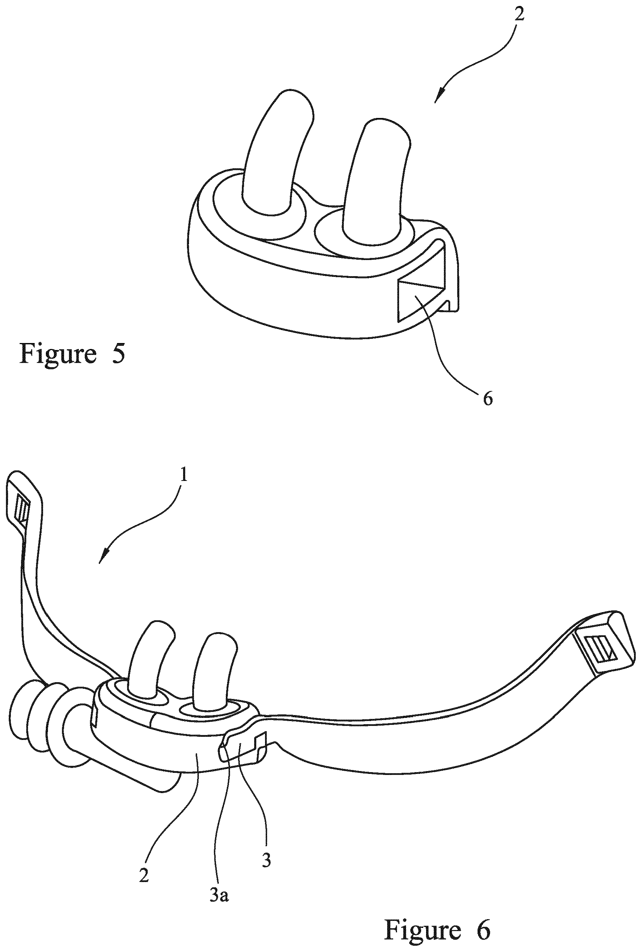

**Figure 5** illustrates a body (2) having openings (6) located at the sides of the body (2) (only one side opening is shown; there is a corresponding opening in the opposite side of the body).

**Figure 6** illustrates an assembled device (1) in which the platform extends through side openings in the body (2). As can be seen, the body (2) is fitted over the front wall of the frame (3). In the illustrated embodiment, the body (2) sits in grooves (3a) in the front wall of the front portion of the frame.

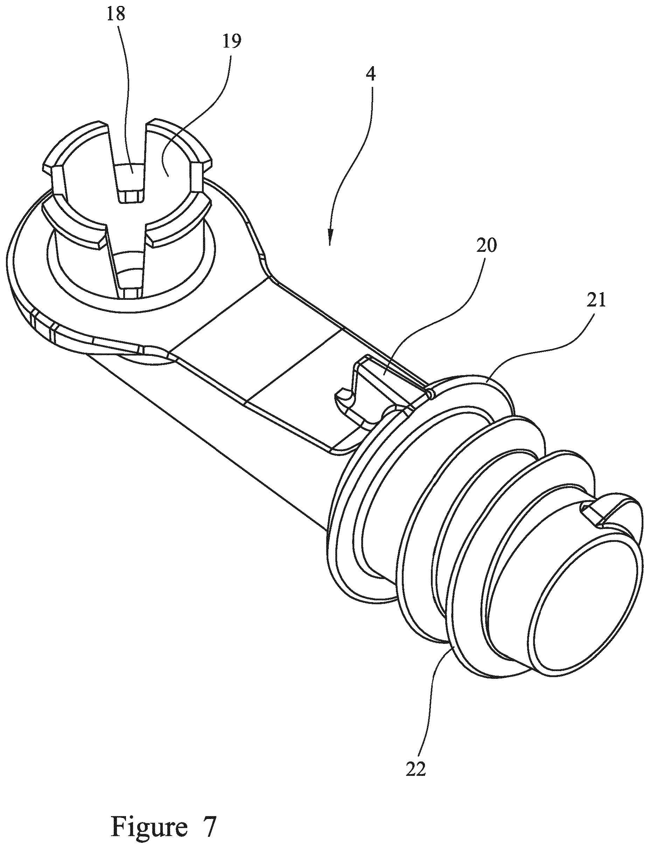

**Figure 7** illustrates an inlet conduit (4) in accordance with the present disclosure. In the embodiment shown, the inlet conduit (4) comprises a connector (18) at a first end, which connector (18) is adapted to extend through the base opening of the body and the inlet opening of the support platform, clamping the base wall of the body and the support platform together. The connector (18) comprises lugs (19) for a permanent snap-fit connection to the top of the annular wall of the platform. The inlet conduit (4) further comprises a projection (20) for engagement with a recess in the frame or body, so as to lock the inlet conduit in a fixed position on the frame or body. The projection depends from an annular projection (21) on the inlet conduit. The second end of the inlet conduit (4) is adapted for connection with a gases supply tube. In the illustrated embodiment, a screw thread (22) is provided at the second end of the inlet conduit, to secure a gases supply tube to the device of the present disclosure.

**Figure 8** illustrates a device (1) in accordance with the present disclosure attached to a head strap (23) and a gases supply tube (24). A clip (25) may be provided, for example to allow the tube (24) to be connected for example to clothing or bedding.

## Claims

1. A respiratory interface device (1) comprising a body (2), a frame (3) and an inlet conduit (4), wherein:
the frame (3) comprises: a front portion (10) having a front wall (10a) and two side walls (10b); side arms (12); and a support platform (11) extending rearwardly from said front wall, the support platform (11) having an inlet opening (13) formed therein;
the body (2) comprises at least one nasal outlet (5) for interfacing with a user's nostril in use, at least one opening (6) through which the support platform extends, and a base wall having a base opening (7) therein; and wherein
the inlet conduit (4) comprises, at a first end thereof, a connector (18) that extends through the base opening (7) of the body (2) and the inlet opening (13) of the support platform (11), and clamps the base wall of the body (2) and the support platform (11) together, a chamber being defined between the support platform (11) and the body (2), which chamber provides fluid communication between the inlet conduit (4) and the one or more nasal outlets (5).

2. A respiratory interface device (1) as claimed in claim 1, wherein the at least one opening (6) through which the support platform (11) extends is selected from a front opening in a front wall of the body and a pair of side openings in side walls of the body.

3. A respiratory interface device (1) as claimed claim 1 or claim 2, wherein the side walls (10b) of the front portion (10) of the frame (3) are separated from the platform (11).

4. A respiratory interface device (1) as claimed in any one of the preceding claims, wherein the support platform (11) comprises a forward wall (14), which forward wall (14) is spaced from the front wall (10a) of the frame (3) such that a channel is formed between said forward (14) and front (10a) walls, and optionally wherein the support platform (11) comprises any of side walls and a rear wall.

5. A respiratory interface device (1) as claimed in any one of the preceding claims, wherein a rear edge (11a) of the support platform (11), or a rear wall of the support platform (11), is shaped to correspond with the shape of a patient's upper lip, curving inwardly from its outer edges.

6. A respiratory interface device (1) as claimed in any one of the preceding claims, wherein the inlet opening (13) in the support platform (11) is circular in shape and, optionally, wherein the support platform (11) comprises an annular wall (13a) extending upwardly around the inlet opening (13).

7. A respiratory interface device (1) as claimed in any one of the preceding claims, wherein the side arms (12) are contoured to correspond with the contours of a patient's face and, optionally, wherein each side arm (12) comprises means (17) for attachment to headgear.

8. A respiratory interface device (1) as claimed in any one of the preceding claims, comprising means for holding the inlet conduit (4) in a fixed position on the front portion of the frame (3), wherein: the inlet conduit (4) comprises a projection (20) and each side wall (10b) of the front portion (10) of the frame (3) comprises a corresponding recess (16); or the inlet conduit (4) comprises a recess and each side wall (10b) of the front portion (10) of the frame (3) comprises a corresponding projection.

9. A respiratory interface device (1) as claimed in any one of the preceding claims, wherein the base opening (7) in the base wall of the body (2) is arranged to correspond with the inlet opening (13) of the support platform (11); and the body (2) further comprises a front wall that comprises the opening (6) through which the platform extends, a rear wall (8), side walls and a top wall that comprises the one or more nasal outlets (5) for interfacing with a patient's nostrils in use.

10. A respiratory interface device (1) as claimed in any one of the preceding claims, wherein the body (2) comprises an annular exteriorly raised ring (7a), around the base opening (7).

11. A respiratory interface device (1) as claimed in any one of the preceding claims, wherein: each of the one or more nasal outlets (5) is curved in shape, curving rearwardly and inwardly from its base with respect to the front and sides of the body and optionally each of the one or more nasal outlets is of uniform cross-section and/or has a uniform wall thickness along their length; and optionally wherein an area (9) around the base of each of the one or more nasal outlets (5) is thinner than the rest of the body (2).

12. A respiratory interface device (1) as claimed in claim 9, wherein the rear wall (8) of the body (2) extends beyond the base wall of the body (2), providing an increased area for contact with a patient's upper lip, and optionally wherein the rear wall (8) of the body (2) is shaped to correspond with the shape of a patient's upper lip, curving inwardly from its outer edges.

13. A respiratory interface device (1) as claimed in any one of the preceding claims, wherein the body (2) is made from a pliable material, for example, silicone, and the frame (3) is made from a flexible plastics material, for example, a thermoplastic synthetic polymer, for example polypropylene or polyethylene.

14. A respiratory interface device (1) as claimed in any one of the preceding claims, wherein the inlet conduit (4) is in the form of an elbow and has, at a first end thereof, a connector (18) extending upwardly from the inlet conduit (4), for connecting to the platform (11) of the frame (3), which connector (18) has a shape complementary to the shape of the inlet opening (13); and optionally wherein the connector (18) is provided with means for a permanent snap-fit to the platform, for example wherein the connector (18) is provided with one or more lugs (19) adapted for a snap-fit with the platform (11).

15. A respiratory interface device (1) as claimed in any one of the preceding claims, wherein the inlet conduit (4) is secured to the platform (11) in a rotatable and non-removable manner.

16. A respiratory interface device kit comprising:
**a** frame (3),
a body (2), and an inlet conduit (4), as separate parts or fully assembled parts, which parts (2, 3, 4) form, when fully assembled, a respiratory interface device (1) as claimed in any one of the preceding claims; and
instructions for assembly and/or use, the kit optionally comprising two or more of said bodies (2) in different sizes.

17. A respiratory interface device (1) comprising a body (2), a frame (3) and an inlet conduit (4), wherein:
the body (2) comprises front, rear (8), base, side and top walls, the front wall having a front opening (6), the base wall having a base opening (7), and the top wall having one or more nasal outlets (5) for interfacing with a patient's nostrils;
the frame (3) comprises a front portion (10) having a front wall (10a) and two side walls (10b); side arms (12); and a support platform (11) extending rearwardly from said front wall (10a), the support platform (11) having an inlet opening (13) formed therein;
each side wall (10b) of the front portion (10) of the frame (3) is separated from the support platform (11);
the base opening (7) of the body (2) is arranged to correspond with the inlet opening (13) of the support platform (11);
the inlet conduit (4) has, at a first end thereof, a connector (18);
the support platform (11) extends through the front opening (6) in the front wall of the body (2); and
the inlet conduit (4) extends through the base opening (7) in the base wall of the body (2) and the inlet opening (13) of the support platform (11), clamping the body (2) and the support platform (11) together.

## Patentansprüche

1. Beatmungsinterfacevorrichtung (1), umfassend einen Körper (2), einen Rahmen (3) und eine Einlassleitung (4), wobei:
der Rahmen (3) Folgendes umfasst: einen vorderen Abschnitt (10) mit einer vorderen Wand (10a) und zwei Seitenwänden (10b); Seitenarme (12); und eine Stützplattform (11), die sich von der vorderen Wand nach hinten erstreckt, wobei die Stützplattform (11) eine darin gebildete Einlassöffnung (13) aufweist;
der Körper (2) mindestens einen Nasenauslass (5) zum Bilden einer Interface mit einem Nasenloch eines Benutzers bei Verwendung, mindestens eine Öffnung (6), durch die sich die Stützplattform erstreckt, und eine Bodenwand mit einer Bodenöffnung (7) darin umfasst; und wobei
die Einlassleitung (4) an einem ersten Ende davon einen Verbinder (18) umfasst, der sich durch die Bodenöffnung (7) des Körpers (2) und die Einlassöffnung (13) der Stützplattform (11) erstreckt und die Bodenwand des Körpers (2) und die Stützplattform (11) zusammenklemmt, wobei eine Kammer zwischen der Stützplattform (11) und dem Körper (2) definiert ist, wobei die Kammer eine Fluidverbindung zwischen der Einlassleitung (4) und dem einen oder den mehreren Nasenauslässen (5) bereitstellt.

2. Beatmungsinterfacevorrichtung (1) nach Anspruch 1, wobei die mindestens eine Öffnung (6), durch die sich die Stützplattform (11) erstreckt, aus einer vorderen Öffnung in einer vorderen Wand des Körpers und einem Paar von Seitenöffnungen in Seitenwänden des Körpers ausgewählt ist.

3. Beatmungsinterfacevorrichtung (1) nach Anspruch 1 oder Anspruch 2, wobei die Seitenwände (10b) des vorderen Abschnitts (10) des Rahmens (3) von der Plattform (11) getrennt sind.

4. Beatmungsinterfacevorrichtung (1) nach einem der vorhergehenden Ansprüche, wobei die Stützplattform (11) eine vorwärtige Wand (14) umfasst, wobei die vorwärtige Wand (14) von der vorderen Wand (10a) des Rahmens (3) beabstandet ist, sodass ein Kanal zwischen der vorwärtigen (14) und der vorderen (10a) Wand gebildet ist, und wobei die Stützplattform (11) optional eine beliebige von Seitenwänden und einer hinteren Wand umfasst.

5. Beatmungsinterfacevorrichtung (1) nach einem der vorhergehenden Ansprüche, wobei eine hintere Kante (11a) der Stützplattform (11) oder eine hintere Wand der Stützplattform (11) so geformt ist, dass sie der Form einer Oberlippe eines Patienten entspricht und sich von ihren äußeren Kanten nach innen krümmt.

6. Beatmungsinterfacevorrichtung (1) nach einem der vorhergehenden Ansprüche, wobei die Einlassöffnung (13) in der Stützplattform (11) kreisförmig ist und wobei die Stützplattform (11) optional eine ringförmige Wand (13a) umfasst, die sich nach oben um die Einlassöffnung (13) erstreckt.

7. Beatmungsinterfacevorrichtung (1) nach einem der vorhergehenden Ansprüche, wobei die Seitenarme (12) so konturiert sind, dass sie den Konturen eines Gesichts eines Patienten entsprechen, und wobei optional jeder Seitenarm (12) Mittel (17) zum Anbringen an einer Kopfbedeckung umfasst.

8. Beatmungsinterfacevorrichtung (1) nach einem der vorhergehenden Ansprüche, umfassend Mittel zum Halten der Einlassleitung (4) in einer festen Position an dem vorderen Abschnitt des Rahmens (3), wobei: die Einlassleitung (4) einen Vorsprung (20) umfasst und jede Seitenwand (10b) des vorderen Abschnitts (10) des Rahmens (3) eine entsprechende Aussparung (16) umfasst; oder die Einlassleitung (4) eine Aussparung umfasst und jede Seitenwand (10b) des vorderen Abschnitts (10) des Rahmens (3) einen entsprechenden Vorsprung umfasst.

9. Beatmungsinterfacevorrichtung (1) nach einem der vorhergehenden Ansprüche, wobei die Bodenöffnung (7) in der Bodenwand des Körpers (2) angeordnet ist, um der Einlassöffnung (13) der Stützplattform (11) zu entsprechen; und der Körper (2) ferner eine vordere Wand, welche die Öffnung (6) umfasst, durch die sich die Plattform erstreckt, eine hintere Wand (8), Seitenwände und eine obere Wand umfasst, die den einen oder die mehreren Nasenauslässe (5) zum Bilden einer Interface mit Nasenlöchern eines Patienten bei Verwendung umfasst.

10. Beatmungsinterfacevorrichtung (1) nach einem der vorhergehenden Ansprüche, wobei der Körper (2) einen ringförmigen, nach außen erhöhten Ring (7a) um die Bodenöffnung (7) umfasst.

11. Beatmungsinterfacevorrichtung (1) nach einem der vorhergehenden Ansprüche, wobei: jeder des einen oder der mehreren Nasenauslässe (5) eine gekrümmte Form hat, die von ihrem Boden in Bezug auf die Vorderseite und die Seiten des Körpers nach hinten und nach innen gekrümmt ist, und wobei optional jeder von dem einen oder den mehreren Nasenauslässen einen gleichmäßigen Querschnitt hat und/oder eine gleichmäßige Wanddicke entlang seiner Länge aufweist; und wobei optional ein Bereich (9) um den Boden jedes von dem einen oder den mehreren Nasenauslässen (5) dünner als der Rest des Körpers (2) ist.

12. Beatmungsinterfacevorrichtung (1) nach Anspruch 9, wobei sich die hintere Wand (8) des Körpers (2) über die Bodenwand des Körpers (2) hinaus erstreckt und einen vergrößerten Bereich für den Kontakt mit einer Oberlippe eines Patienten bereitstellt und wobei die hintere Wand (8) des Körpers (2) optional so geformt ist, dass sie der Form einer Oberlippe eines Patienten entspricht und sich von ihren äußeren Kanten nach innen krümmt.

13. Beatmungsinterfacevorrichtung (1) nach einem der vorhergehenden Ansprüche, wobei der Körper (2) aus einem biegsamen Material, zum Beispiel Silikon, hergestellt ist und der Rahmen (3) aus einem flexiblen Kunststoffmaterial, zum Beispiel einem thermoplastischen synthetischen Polymer, zum Beispiel Polypropylen oder Polyethylen, hergestellt ist.

14. Beatmungsinterfacevorrichtung (1) nach einem der vorhergehenden Ansprüche, wobei die Einlassleitung (4) die Form eines Ellbogens hat und an einem ersten Ende davon einen Verbinder (18) aufweist, der sich von der Einlassleitung (4) nach oben erstreckt, um mit der Plattform (11) des Rahmens (3) verbunden zu werden, wobei der Verbinder (18) eine Form aufweist, die zu der Form der Einlassöffnung (13) komplementär ist; und wobei der Verbinder (18) optional mit Mitteln für eine dauerhafte Schnappverbindung mit der Plattform bereitgestellt ist, wobei der Verbinder (18) zum Beispiel mit einem oder mehreren Ansätzen (19) bereitgestellt ist, die für eine Schnappverbindung mit der Plattform (11) angepasst sind.

15. Beatmungsinterfacevorrichtung (1) nach einem der vorhergehenden Ansprüche, wobei die Einlassleitung (4) auf eine drehbare und nicht entfernbare Weise an der Plattform (11) befestigt ist.

16. Beatmungsinterfacevorrichtungskit, umfassend:
einen Rahmen (3), einen Körper (2) und eine Einlassleitung (4) als separate Teile oder vollständig zusammengebaute Teile, wobei die Teile (2, 3, 4), wenn sie vollständig zusammengebaut sind, eine Beatmungsinterfacevorrichtung (1) nach einem der vorhergehenden Ansprüche bilden; und
Anweisungen für Zusammenbau und/oder Verwendung, wobei das Kit optional zwei oder mehr der Körper (2) in verschiedenen Größen umfasst.

17. Beatmungsinterfacevorrichtung (1), umfassend einen Körper (2), einen Rahmen (3) und eine Einlassleitung (4), wobei:
der Körper (2) eine vordere, hintere (8), Boden-, Seiten- und obere Wand umfasst, wobei die vordere Wand eine vordere Öffnung (6) aufweist, die Bodenwand eine Bodenöffnung (7) aufweist und die obere Wand einen oder mehrere Nasenauslässe (5) zum Bilden einer Interface mit Nasenlöchern eines Patienten aufweist;
der Rahmen (3) einen vorderen Abschnitt (10) mit einer vorderen Wand (10a) und zwei Seitenwänden (10b); Seitenarme (12); und eine Stützplattform (11) umfasst, die sich von der vorderen Wand (10a) nach hinten erstreckt, wobei die Stützplattform (11) eine darin gebildete Einlassöffnung (13) aufweist;
jede Seitenwand (10b) des vorderen Abschnitts (10) des Rahmens (3) von der Stützplattform (11) getrennt ist;
die Bodenöffnung (7) des Körpers (2) angeordnet ist, um der Einlassöffnung (13) der Stützplattform (11) zu entsprechen;
die Einlassleitung (4) an einem ersten Ende davon einen Verbinder (18) aufweist;
sich die Stützplattform (11) durch die vordere Öffnung (6) in der vorderen Wand des Körpers (2) erstreckt; und
sich die Einlassleitung (4) durch die Bodenöffnung (7) in der Bodenwand des Körpers (2) und die Einlassöffnung (13) der Stützplattform (11) erstreckt, wobei der Körper (2) und die Stützplattform (11) zusammenklemmt sind.

## Revendications

1. Dispositif interface respiratoire (1) comprenant un corps (2), un cadre (3) et un conduit d'entrée (4) :
ledit cadre (3) comprenant : une partie frontale (10) comportant une paroi frontale (10a) et deux parois latérales (10b) ; des bras latéraux (12) ; et une plateforme support (11) s'étendant vers l'arrière à partir de ladite paroi frontale, ladite plateforme support (11) comportant une ouverture d'entrée (13) formée en son sein ;
ledit corps (2) comprenant au moins une sortie nasale (5) destinée à un interfaçage avec la narine d'un utilisateur lors de l'utilisation, au moins une ouverture (6) à travers laquelle s'étend la plateforme support, et une paroi de base comportant une ouverture de base (7) en son sein ; et
ledit conduit d'entrée (4) comprenant, au niveau d'une première extrémité de celui-ci, un raccord (18) qui s'étend à travers l'ouverture de base (7) du corps (2) et l'ouverture d'entrée (13) de la plateforme support (11), et serrant la paroi de base du corps (2) et la plateforme support (11) ensemble, une chambre étant définie entre la plateforme support (11) et le corps (2), laquelle chambre assurant une communication fluidique entre le conduit d'entrée (4) et lesdites une ou plusieurs sorties nasales (5).

2. Dispositif interface respiratoire (1) selon la revendication 1, ladite au moins une ouverture (6) à travers laquelle s'étend la plateforme support (11) étant sélectionnée parmi une ouverture frontale dans une paroi frontale du corps et deux ouvertures latérales dans des parois latérales du corps.

3. Dispositif interface respiratoire (1) selon la revendication 1 ou la revendication 2, lesdites parois latérales (10b) de la partie frontale (10) du cadre (3) étant séparées de la plateforme (11).

4. Dispositif interface respiratoire (1) selon l'une quelconque des revendications précédentes, ladite plateforme support (11) comprenant une paroi avant (14), laquelle paroi avant (14) étant espacée de la paroi frontale (10a) du cadre (3) de sorte qu'un canal soit formé entre lesdites parois avant (14) et frontale (10a), et éventuellement ladite plateforme support (11) comprenant l'une quelconque des parois latérales et une paroi arrière.

5. Dispositif interface respiratoire (1) selon l'une quelconque des revendications précédentes, un bord arrière (11a) de la plateforme support (11), ou une paroi arrière de la plateforme support (11), étant formé de manière à correspondre à la forme de la lèvre supérieure d'un patient, incurvée vers l'intérieur à partir de ses bords extérieurs.

6. Dispositif interface respiratoire (1) selon l'une quelconque des revendications précédentes, ladite ouverture d'entrée (13) dans la plateforme support (11) étant de forme circulaire et, éventuellement, ladite plateforme support (11) comprenant une paroi annulaire (13a) s'étendant vers le haut autour de l'ouverture d'entrée (13).

7. Dispositif interface respiratoire (1) selon l'une quelconque des revendications précédentes, lesdits bras latéraux (12) étant profilés de manière à correspondre aux contours du visage d'un patient et, éventuellement, chaque bras latéral (12) comprenant un moyen (17) pour la fixation à un casque.

8. Dispositif interface respiratoire (1) selon l'une quelconque des revendications précédentes, comprenant un moyen pour maintenir le conduit d'entrée (4) dans une position fixe sur la partie avant du cadre (3) : ledit conduit d'entrée (4) comprenant une saillie (20) et chaque paroi latérale (10b) de la partie frontale (10) du cadre (3) comprenant un évidement correspondant (16) ; ou ledit conduit d'entrée (4) comprenant un évidement et chaque paroi latérale (10b) de la partie frontale (10) du cadre (3) comprenant une saillie correspondante.

9. Dispositif interface respiratoire (1) selon l'une quelconque des revendications précédentes, ladite ouverture de base (7) dans la paroi de base du corps (2) étant agencée de manière à correspondre à l'ouverture d'entrée (13) de la plateforme support (11) ; et ledit corps (2) comprenant en outre une paroi frontale qui comprend l'ouverture (6) à travers laquelle s'étend la plateforme, une paroi arrière (8), des parois latérales et une paroi supérieure qui comprend lesdites une ou plusieurs sorties nasales (5) pour un interfaçage avec les narines d'un patient lors de l'utilisation.

10. Dispositif interface respiratoire (1) selon l'une quelconque des revendications précédentes, ledit corps (2) comprenant une bague annulaire surélevée extérieurement (7a), autour de l'ouverture de base (7).

11. Dispositif interface respiratoire (1) selon l'une quelconque des revendications précédentes : chacune desdites une ou plusieurs sorties nasales (5) présentant une forme incurvée, incurvée vers l'arrière et vers l'intérieur à partir de sa base par rapport à la partie frontale et aux côtés du corps et éventuellement chacune desdites une ou plusieurs sorties nasales présentant une section transversale uniforme et/ou présentant une épaisseur de paroi uniforme sur leur longueur ; et éventuellement une zone (9) autour de la base de chacune desdites une ou plusieurs sorties nasales (5) étant plus mince que le reste du corps (2).

12. Dispositif interface respiratoire (1) selon la revendication 9, ladite paroi arrière (8) du corps (2) s'étendant au-delà de la paroi de base du corps (2), fournissant une zone accrue pour un contact avec la lèvre supérieure d'un patient, et éventuellement ladite paroi arrière (8) du corps (2) étant formée de manière à correspondre à la forme de la lèvre supérieure d'un patient, incurvée vers l'intérieur à partir de ses bords extérieurs.

13. Dispositif interface respiratoire (1) selon l'une quelconque des revendications précédentes, ledit corps (2) étant fabriqué à partir d'un matériau pliable, par exemple, du silicone, et ledit cadre (3) étant fabriqué à partir d'un matériau plastique flexible, par exemple, un polymère synthétique thermoplastique, par exemple, du polypropylène ou du polyéthylène.

14. Dispositif interface respiratoire (1) selon l'une quelconque des revendications précédentes, ledit conduit d'entrée (4) étant sous la forme d'un coude et comportant, au niveau d'une première extrémité de celui-ci, un raccord (18) s'étendant vers le haut à partir du conduit d'entrée (4), pour un raccordement à la plateforme (11) du cadre (3), lequel raccord (18) comportant une forme complémentaire à la forme de l'ouverture d'entrée (13) ; et éventuellement ledit raccord (18) étant pourvu d'un moyen pour un encliquetage permanent sur la plateforme, par exemple, ledit raccord (18) étant pourvu d'une ou plusieurs pattes (19) adaptées à un encliquetage avec la plateforme (11).

15. Dispositif interface respiratoire (1) selon l'une quelconque des revendications précédentes, ledit conduit d'entrée (4) étant fixé à la plateforme (11) de manière rotative et non amovible.

16. Kit de dispositif interface respiratoire comprenant :
un cadre (3) ;
un corps (2) ;
et un conduit d'entrée (4), en tant que pièces distinctes ou que pièces entièrement assemblées, lesquelles pièces (2, 3, 4) forment, lorsqu'elles sont entièrement assemblées, un dispositif interface respiratoire (1) selon l'une quelconque des revendications précédentes ; et
instructions d'assemblage et/ou d'utilisation, le kit comprenant éventuellement deux desdits corps (2) ou plus de tailles différentes.

17. Dispositif interface respiratoire (1) comprenant un corps (2), un cadre (3) et un conduit d'entrée (4) :
ledit corps (2) comprenant des parois frontale, arrière (8), de base, latérales et supérieure, ladite paroi frontale comportant une ouverture frontale (6), ladite paroi de base comportant une ouverture de base (7), et ladite paroi supérieure comportant une ou plusieurs sorties nasales (5) pour un interfaçage avec les narines d'un patient ;
ledit cadre (3) comprenant : une partie frontale (10) comportant une paroi frontale (10a) et deux parois latérales (10b) ; des bras latéraux (12) ; et une plateforme support (11) s'étendant vers l'arrière à partir de ladite paroi frontale (10a), ladite plateforme support (11) comportant une ouverture d'entrée (13) formée en son sein ;
chaque paroi latérale (10b) de la partie frontale (10) du cadre (3) étant séparée de la plateforme support (11) ;
ladite ouverture de base (7) du corps (2) étant agencée de manière à correspondre à l'ouverture d'entrée (13) de la plateforme support (11) ;
ledit conduit d'entrée (4) comportant, au niveau d'une première extrémité de celui-ci, un raccord (18) ;
ladite plateforme support (11) s'étendant à travers l'ouverture frontale (6) dans la paroi frontale du corps (2) ; et
ledit conduit d'entrée (4) s'étendant à travers l'ouverture de base (7) dans la paroi de base du corps (2) et ladite ouverture d'entrée (13) de la plateforme support (11), serrant le corps (2) et la plateforme support (11) ensemble.
